**Europäisches Patentamt**

**European Patent Office**

**Office européen des brevets**

(11) Numéro de publication: **0 320 046**
**A1**

## (12) DEMANDE DE BREVET EUROPEEN

(21) Numéro de dépôt: **88202717.0**

(22) Date de dépôt: **29.11.88**

(51) Int. Cl.4: **C07C 59/01 , C07C 51/44 ,
B01D 1/22**

(30) Priorité: **10.12.87 FR 8717379**

(43) Date de publication de la demande:
**14.06.89 Bulletin 89/24**

(84) Etats contractants désignés:
**AT BE CH DE ES FR GB GR IT LI LU NL SE**

(71) Demandeur: **SOLVAY & Cie (Société Anonyme)**
**Rue du Prince Albert, 33**
**B-1050 Bruxelles(BE)**

(72) Inventeur: **Blondeel, Georges**
**Hoezenstraat 10**
**B-9300 Aalst(BE)**

(54) **Procédé d'obtention d'acide D(-)-3-hydroxybutanoique de grande pureté.**

(57) L'hydrolysat de poly-bêta-hydroxybutyrates est soumis à une distillation suivant la technique de l'évaporation à film mince à une température comprise entre 70 et 140° C et à une pression absolue comprise entre 0,2 et 15 mbars.

Ce procédé permet d'obtenir l'acide D(-)-3-hydroxybutanoïque d'une très grande pureté utilisable notamment en pharmacie.

EP 0 320 046 A1

Figure 1

## Procédé d'obtention d'acide D(-)-3-hydroxybutanoïque de grande pureté

La présente invention concerne un procédé d'obtention d'acide D(-)-3-hydroxybutanoïque de grande pureté qui fait intervenir une opération de distillation suivant la technique de l'évaporation à film mince d'un hydrolysat obtenu à partir de poly-bêta-hydroxybutyrates extraits d'une biomasse.

L'acide D(-)-3-hydroxybutanoïque peut être obtenu selon plusieurs types de procédés comprenant notamment des procédés de synthèse purement chimiques à partir de composés simples et des procédés faisant intervenir des cellules vivantes. Parmi ces derniers procédés, un type particulier consiste à mettre en oeuvre des microorganismes qui synthétisent des polymères tels que les poly-bêta-hydroxybutyrates. Dans ce cas, les poly-bêta-hydroxybutyrates peuvent être dégradés par des procédés d'hydrolyse, comme notamment celui décrit dans le brevet européen 0043620 au nom de SOLVAY & Cie, après extraction éventuelle des poly-bêta-hydroxybutyrates de la biomasse tel que décrit dans le brevet européen 0014490 également au nom de SOLVAY & Cie.

Selon les conditions particulières d'hydrolyse choisies, l'hydrolysat obtenu contient une concentration plus ou moins grande d'acide D(-)-3-hydroxybutanoïque, qui peut atteindre, voire dépasser, 500 g/kg de l'hydrolysat total, lorsque les conditions d'hydrolyse sont ajustées pour optimaliser l'obtention de cet acide sous forme monomère.

A partir de cet hydrolysat, l'acide D(-)-3-hydroxybutanoïque peut en principe être isolé et purifié selon des techniques telles que celles notamment décrites dans le brevet européen 0043620 précité. Il existe toutefois un problème pratique si l'on désire obtenir à partir d'un hydrolysat de poly-bêta-hydroxybutyrates de l'acide D(-)-3- hydroxybutanoïque de grande pureté avec un rendement industriellement acceptable.

En effet, aucune méthode physique industrielle de séparation connue n'a donné jusqu'à présent un résultat acceptable et, plus particulièrement, les opérations de distillation réalisées jusqu'à présent mènent à une oligomérisation et/ou dégradation de l'acide D(-)-3-hydroxybutanoïque en acide crotonique.

On a maintenant trouvé un procédé de séparation de type physique qui permet d'éviter ces inconvénients et qui permet d'obtenir l'acide D(-)-3-hydroxybutanoïque avec une grande pureté.

La présente invention concerne à cet effet un procédé pour l'obtention d'acide D(-)-3-hydroxybutanoïque de grande pureté à partir d'un hydrolysat de poly-bêta-hydroxybutyrates, provenant d'une biomasse, dans lequel cet hydrolysat, contenant au moins 500 g/kg d'acide D(-)-3-hydroxybutanoïque monomère, est soumis à une opération de distillation suivant la technique de l'évaporation à film mince à une température de l'évaporateur comprise entre 70 et 140°C et à une pression absolue comprise entre 0,2 et 15 mbars.

Par technique de l'évaporation à film mince, on entend toute technique visant à réduire à un minimum la durée d'échauffement, tout en créant le moins possible d'hétérogénéités thermiques, et réalisant des transferts thermiques rapides. La technique de l'évaporation à film mince inclut la technique de la distillation moléculaire.

Le temps de séjour des produits dans l'appareil d'évaporation dépend directement du rapport entre le débit d'alimentation de l'appareil et l'aire de la surface la plus chaude (surface d'évaporation) de l'appareil. Généralement, ce rapport est inférieur à 7,000 g/m$^2$.h; habituellement, il est compris entre 200 et 5.000, de manière préférée, entre 250 et 4.000 et, de manière particulièrement préférée, entre 300 et 3.500 g/m$^2$.h.

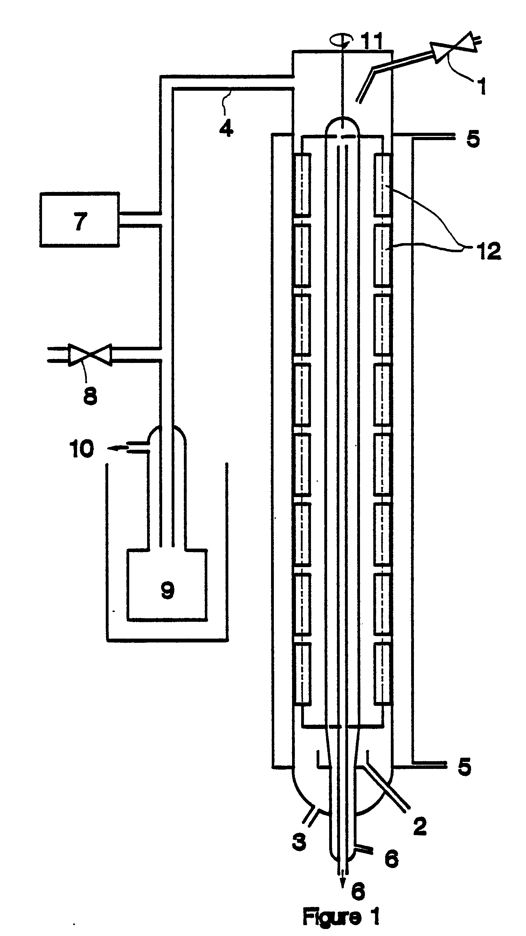

De préférence, l'hydrolysat mis en oeuvre dans le procédé de l'invention contient entre 700 et 900 g/kg d'acide D(-)-3-hydroxybutanoïque. L'évaporation à film mince peut être réalisée dans tout appareil permettant d'effectuer une telle opération. Habituellement, on opère dans des évaporateurs à film utilisés dans le domaine d'application de la technique de l'évaporation à film mince ou dans des appareils utilisés dans le domaine de la distillation moléculaire. Préférentiellement, l'opération de distillation est effectuée avec des appareils à film ruisselant ou à film agité tels que les appareils à pales fixes, à pales mobiles et à frotteurs mobiles y compris les appareils opérant par centrifugation équipés d'un évaporateur et d'un condenseur. Le schéma de l'évaporateur à film agité au moyen de cylindres racleurs ayant été utilisé avec de bons résultats dans les exemples est illustré par la figure 1 dans laquelle les éléments constitutifs sont donnés par les repères suivants :

1 : vanne d'alimentation en hydrolysat
2 : sortie fraction légère
3 : sortie fraction lourde
4 : sortie vapeur
5 : chauffage extérieur (surface la plus chaude on surface d'évaporation)
6 : chauffage intérieur (condenseur)
7 : manomètre
8 : vanne permettant de régler le vide

9 : piège froid à - 80° C

10 : pompe à vide

11 : moteur pour l'entraînement des cylindres

12 : cylindres racleurs (évaporateur).

La température de l'évaporateur, à laquelle la distillation est effectuée, se situe entre 85 et 125° C. La température du condenseur est habituellement comprise entre 30 et 60° C.

La distillation est réalisée à une pression de préférence comprise entre 0,4 et 5 mbars.

Le débit d'alimentation de l'appareil de distillation peut varier selon le type et les dimensions de l'appareil utilisé. Pour l'appareil schématisé et utilisé dans les exemples, des débits d'alimentation compris entre 200 et 800 g/h ont donné de bons résultats.

Outre l'acide D(-)-3-hydroxybutanoïque, l'hydrolysat mis en oeuvre peut contenir notamment des oligomères de cet acide, de l'eau et des traces d'acide crotonique. Généralement, les oligomères sont des di-, tri-, tétra- et pentamères de l'acide monomère.

Les hydrolysats, ayant donné de bons résultats lorsqu'ils sont mis en oeuvre selon le procédé de l'invention, contiennent outre l'acide D(-)-3-hydroxybutanoïque, de 100 à 200 g/kg de dimères, de 10 à 50 g/kg de trimères, des traces de tétramères et d'acide crotonique, ainsi que de 0 à 50 g/kg d'eau.

L'hydrolysat de poly-bêta-hydroxybutyrates mis en oeuvre dans le procédé de l'invention peut provenir de toute biomasse produisant le polymère en question et l'extraction et/ou la solubilisation du polymère peuvent être effectuées par n'importe quel moyen, tel que notamment par un solvant organique ou inorganique connu à cet effet ou par de l'hypochlorite de sodium. De bons résultats ont été obtenus avec des hydrolysats obtenus selon le procédé qui fait l'objet du brevet européen 0043620.

Habituellement, l'hydrolysat de poly-bêta-hydroxybutyrates mis en oeuvre dans le procédé selon l'invention est soluble dans l'eau.

Par acide D(-)-3-hydroxybutanoïque de grande pureté, on entend désigner l'acide D(-)-3-hydroxybutanoïque monomère contenant moins de 2 % en poids d'impuretés.

De préférence, un tel acide D(-)-3-hydroxybutanoïque contient en outre moins de 3 g/kg d'acide crotonique. Enfin, de façon tout particulièrement préférée, on vise par le procédé de l'invention l'obtention d'acide D(-)-3-hydroxybutanoïque contenant au total moins de 1 % en poids d'impuretés et en particulier moins de 2,5 g/kg d'acide crotonique.

L'acide D(-)-3-hydroxybutanoïque de très grande pureté obtenu selon le procédé de l'invention peut être mis en oeuvre dans toute utilisation chimique et/ou pharmaceutique requérant des produits de grande pureté et notamment pour la fabrication de sels de cet acide destinés à être administrés à des patients par voie intraveineuse comme décrit dans le brevet européen 0087192 au nom de SOLVAY & Cie.

L'invention est illustrée par les exemples qui suivent.

## Exemple 1

La distillation a lieu dans un évaporateur à film raclé, équipé de rouleaux en teflon tel qu'illustré à la figure 1, avec une surface chauffante d'environ 0,16 m² et avec une surface du condenseur interne concentrique d'environ 0,07 m². La distance entre l'évaporateur et le condenseur est comprise entre 2,5 et 3,0 cm.

La température de l'évaporateur est réglée à 107° C, la température du condenseur à 50° C. La pression absolue est de 3,3 mbars.

Le débit d'alimentation s'élève à 530 g.h$^{-1}$.

Le rapport entre le débit d'alimentation et l'aire de la surface la plus chaude est donc de 3313 g/m².h.

L'hydrolysat de poly-bêta-hydroxybutyrates mis en oeuvre provient de l'extraction d'une biomasse issue d'Alcaligenes eutrophus.

La composition de l'hydrolysat mis en oeuvre et du produit obtenu après la distillation est décrite dans le tableau 1.

Tableau 1

| Composition en g/kg | Hydrolysat mis en oeuvre | Fraction légère | Fraction lourde |
|---|---|---|---|
| acide D(-)-3-hydroxybutanoïque | 721 | 985 | 375 |
| dimère de cet acide | 195 | 13 | 477 |
| trimère de cet acide | 46 | < 1 | 115 |
| tétramère de cet acide | 10 | < 1 | 30 |
| acide crotonique | 1,9 | 1,6 | < 0,1 |
| eau | 10 | N.D. | N.D. |
| N.D. : non déterminé. | | | |

Le débit de "légers" s'élève à 278 g.h$^{-1}$, le débit de "lourds" à 226 g.h$^{-1}$.

Exemple 2

La distillation a lieu dans l'évaporateur tel que décrit à l'exemple 1.

La température de l'évaporateur est réglée à 97°C, la température du condenseur à 50°C. La pression absolue est de 2,5 mbars.

Le débit d'alimentation s'élève à 280 g.h$^{-1}$.

Le rapport entre le débit d'alimentation et l'aire de la surface la plus chaude est donc de 1750 g/m$^2$.h.

La composition de l'hydrolysat mis en oeuvre et du produit obtenu après distillation est décrite dans le tableau 2.

Tableau 2

| Composition en g/kg | Hydrolysat mis en oeuvre | Fraction légère | Fraction lourde |
|---|---|---|---|
| acide D(-)-3-hydroxybutanoïque | 721 | 994 | 375 |
| dimère | 195 | 6 | 475 |
| trimère | 46 | < 0,1 | 115 |
| tétramère | 10 | < 0,1 | 31 |
| acide crotonique | 1,9 | < 1 | < 1 |
| eau | 10 | N.D. | N.D. |
| N.D. : non déterminé. | | | |

Le débit de "légers" s'élève à 157 g.h$^{-1}$, le débit de "lourds" à 120 g.h$^{-1}$.

Exemple 3

La distillation a lieu dans l'évaporateur tel que décrit à l'exemple 1.

La température de l'évaporateur est réglée à 107°C, la température du condenseur à 50°C. La pression absolue est de 5,1 mbars.

La débit d'alimentation s'élève à 274 g.h$^{-1}$.

Le rapport entre le débit d'alimentation et l'aire de la surface la plus chaude est donc de 1713 g/m$^2$.h.

La composition de l'hydrolysat mis en oeuvre et du produit obtenu après la distillation est décrite dans le tableau 3.

Tableau 3

| Composition en g/kg | Hydrolysat mis en oeuvre | Fraction légère | Fraction lourde |
|---|---|---|---|
| acide D(-)-3-hydroxybutanoïque | 732 | 991 | 490 |
| dimère | 162 | 5 | 385 |
| trimère | 31 | < 1 | 102 |
| tétramère | 8 | < 1 | 23 |
| acide crotonique | 1,4 | 2,4 | < 1 |
| eau | 44 | N.D. | N.D. |
| N.D. : non déterminé. | | | |

Le débit de "légers" s'élève à 137 g.h$^{-1}$, le débit de "lourds" à 114 g.h$^{-1}$.

Exemple 4R de comparaison

258 g d'un hydrolysat de poly-bêta-hydroxybutyrates contenant 883 g.kg$^{-1}$ d'acide D(-)-3-hydroxybutanoïque, 148 g.kg$^{-1}$ de dimère de cet acide et 1,9 g.kg$^{-1}$ d'acide crotonique, est soumis à une distillation en batch, c'est-à-dire sans addition d'hydrolysat dans le bouilleur, en effet tout l'hydrolysat est introduit dans le bouilleur au début de l'opération.

Les conditions opératoires et les résultats sont rassemblés dans le tableau 4.

Tableau 4

| | Pression mbar | Température du bouilleur °C | Température de tête °C | Poids g | Acide D(-)-3-hydroxybutanoïque g/kg | Dimère g/kg | Acide crotonique g/kg |
|---|---|---|---|---|---|---|---|
| fraction 1 | 0,1 | 102 | 90-93 | 5,0 | 990 | < 1 | 10,4 |
| fraction 2 | 0,1 | 103 | 93-95 | 81,4 | 998 | < 1 | 2,2 |
| fraction 3 | 0,02 | 122 | 100 | 65,4 | 888 | 35,5 | 43,1 |
| résidu | | | | 96,3 | 70 | 560 | 8,8 |

Les quantités retrouvées par rapport aux quantités mises en oeuvre sont donc de 66 % pour l'acide D(-)-3-hydroxybutanoïque, 147 % pour le dimère de cet acide et 795 % pour l'acide crotonique.

On observe une importante oligomérisation et une dégradation en acide crotonique.

Exemple 5R de comparaison

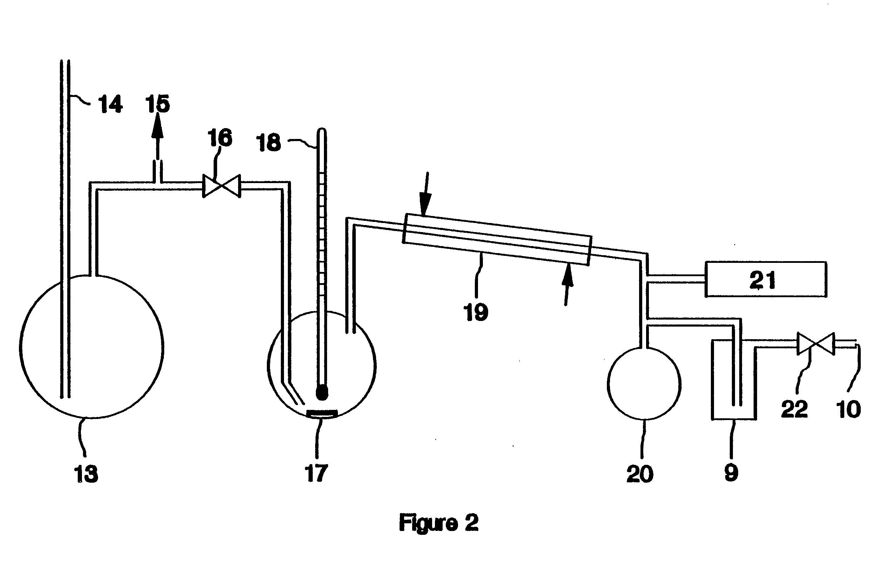

Cet exemple est réalisé dans un appareil de distillation par entraînement à la vapeur, appareil schématisé à la figure 2 dans laquelle les éléments constitutifs sont donnés par les repères suivants :

13 : ballon de 2 l à source de vapeur et muni d'un manteau chauffant (chauffage électrique)

14 : purge de sécurité

15 : purge de réglage

16 : vanne de réglage vapeur

17 : bouilleur de 250 ml muni d'un agitateur magnétique

18 : thermomètre

19 : condenseur

20 : réception du distillat

21 : manomètre

9 : piège froid à -80° C

22 : vanne de réglage (mise à l'air)

10 : pompe à vide.

On introduit 1,7 l d'eau dans le ballon (13) que l'on chauffe à ébullition.

Entre-temps, on introduit dans le bouilleur (17) 124,4 g d'un hydrolysat de poly-bêta-hydroxybutyrates dont la composition est donnée dans le tableau 5.

La température du condenseur (19) est réglée à 50° C.

Quand l'eau est à ébullition dans le ballon (13), la température du bouilleur (17) est amenée à 120° C et la pompe à vide (10) est mise en fonctionnement.

Une pression absolue de 10 mbars est obtenue.

La distillation a lieu, la température du bouilleur (17) est réglée entre 120 et 135° C, la pression absolue maintenue à 10 mbars et la température du condenseur (19) maintenue à 50° C.

Les résultats sont rassemblés dans le tableau 5.

On observe une dégradation en acide crotonique ainsi qu'une concentration élevée de dimère dans le distillat.

Tableau 5

| COMPOSITION | HYDROLYSAT MIS EN OEUVRE 124,4 g | | DISTILLAT ① 64,6 g | | BOUILLEUR ② 48,47 g | | PIEGE * ③ 112,8 g | | BILAN ① + ② + ③ |
|---|---|---|---|---|---|---|---|---|---|
| | g/kg | g.total | g/kg | g.total | g/kg | g.total | g/kg | g.total | g/kg |
| acide D(-)-3-hydroxybutanoïque | 743 | 92,43 | 896 | 57,88 | 194 | 9,4 | 1,5 | 0,17 | 67,45 |
| dimère | 164 | 20,40 | 56 | 3,62 | 438 | 21,22 | - | - | 24,84 |
| trimère | 35 | 4,35 | 6 | 0,39 | 186 | 9,02 | - | - | 9,41 |
| tétramère | 7 | 0,87 | - | | 78 | 3,78 | - | - | 3,78 |
| pentamère | - | - | - | | 23 | 1,12 | - | - | 1,12 |
| acide crotonique | 3,6 | 0,448 | 5,9 | 0,38 | < 1 | - | 6,0 | 0,68 | 1,06 |

* constituant principal = $H_2O$
- non déterminé.

## Revendications

1 - Procédé d'obtention d'acide D(-)-3-hydroxybutanoïque de grande pureté à partir d'un hydrolysat de poly-bêta-hydroxybutyrates, caractérisé en ce que l'hydrolysat, contenant au moins 500 g/kg d'acide D(-)-3-hydroxybutanoïque monomère, est soumis à une opération de distillation suivant la technique de l'évaporation à film mince à une température comprise entre 70 et 140° C et à une pression absolue comprise entre 0,2 et 15 mbars.

2 - Procédé selon la revendication 1, caractérisé en ce que le rapport entre le débit d'alimentation et l'aire de la surface la plus chaude de l'appareil de distillation est inférieur à 7000 g/m².h.

3 - Procédé selon la revendication 1 ou 2, caractérisé en ce que l'hydrolysat contient entre 700 et 900 g/kg d'acide D(-)-3-hydroxybutanoïque et en ce que la distillation est réalisée à une température comprise entre 85 et 125° C et à une pression absolue comprise entre 0,4 et 5 mbars.

4 - Procédé selon la revendication 3, caractérisé en ce que l'hydrolysat contient, outre l'acide D(-)-3-hydroxybutanoïque, de 100 à 200 g/kg de dimère de cet acide, de 10 à 50 g/kg de trimère de cet acide.

5 - Procédé selon l'une quelconque des revendications 1 à 4, caractérisé en ce que l'hydrolysat est soluble dans l'eau.

EP 0 320 046 A1

Figure 1

**Figure 2**

EP 0 320 046 A1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | FR-A-1 536 980 (KOPPERS CO.)<br>* Résumé, point 1; page 2, colonne 2, ligne 48 - page 3, colonne 1, ligne 54; figure 2 *<br>--- | 1 | C 07 C 59/01<br>C 07 C 51/44<br>B 01 D 1/22 |
| Y | US-A-3 382 158 (SMITH)<br>* Revendications 1-6; colonne 2, ligne 45 - colonne 3, ligne 61; figure 2 *<br>--- | 1 | |
| Y | EP-A-0 186 291 (THE MICANITE & INSULATORS CO.)<br>* Page 6, ligne 18 - page 7, ligne 11; figure 1 *<br>--- | 1 | |
| A | FR-A- 915 161 (MÜLLER)<br>* Résumé, points 1,2; page 2, ligne 76 - page 3, ligne 8; figures 1-3 *<br>----- | 1 | |

DOMAINES TECHNIQUES
RECHERCHES (Int. Cl.4)

C 07 C 59/00
C 07 C 51/00
B 01 D 3/00
B 01 D 1/00

Le présent rapport a été établi pour toutes les revendications

| Lieu de la recherche | Date d'achèvement de la recherche | Examinateur |
|---|---|---|
| LA HAYE | 16-03-1989 | KLAG M.J. |